# EUROPÄISCHE PATENTSCHRIFT

(11) **EP 1 190 724 B1**
(45) Veröffentlichungstag und Bekanntmachung des Hinweises auf die Patenterteilung: **25.01.2006**
(21) Anmeldenummer: 00120728.1
(22) Anmeldetag: 22.09.2000
(51) Int. Cl.: A61L 15/44

(54) **Verwendung von Antifibrinolytika für die Präparation und Herstellung von Tupfern, Kompressen oder Pflaster**
Use of antifibrinolytic agents for preparing sponges, compresses or dressings
Utilisation d'agents antifibrinolytique pour la préparation de tampons, compresses ou pansements

(43) Veröffentlichungstag der Anmeldung: 27.03.2002
(73) Patentinhaber: Perlei Medical Produkte GmbH, 02730 Ebersbach/Sachsen (DE)
(72) Erfinder: Boethius, Jörgen, 18363 Täby (SE)
(74) Vertreter: Uhlemann, Henry

(56) Entgegenhaltungen:
- EP-A- 0 090 997
- WO-A-90/13320
- WO-A-93/06855
- WO-A-95/12371
- WO-A-96/40033
- DE-A- 4 115 453
- DATABASE WPI Section Ch, Week 197937 Derwent Publications Ltd., London, GB; Class A96, AN 1979-66949B XP002160780 & JP 54 098091 A (UNITIKA LTD), 2. August 1979 (1979-08-02)

## Beschreibung

Die Erfindung betrifft die Verwendung des Antifibrinolytikums für die Präparation medizinischer Tupfer oder die Herstellung von Tupfern, Kompressen oder Verbandstoffe sowie Tupfer, Kompressen oder Verbandstoffe enthaltend dieses Antifibrinolytikum zur Verbesserung der Blutstillung bei Blutungen. Mit Antifibrinolytika getränkte medizinische Tupfer sind z. Bsp. für den Einsatz bei chirurgischen Operationen bestimmt.

Bekannt ist, zur Stillung von Blutungen Cellulose-, Gelatine- oder Kollagen-Kompressen/Schwämme sowie Fibrinkleber oder Thrombin einzusetzen.

Bei neuro- und mikrochirurgischen Operationen werden zur Blutstillung in physiologischer Kochsalzlösung getränkte Tupfer verwendet. Die Blutstillung ist dabei zeitlich sehr aufwendig und nimmt einen erheblichen Zeitraum in Anspruch.

Der Einsatz bekannter medizinischer Tupfer oder Kompressen ist eingeschränkt, da sie nur eine zeitlich begrenzte Wundstillung und einen begrenzten Wundschutz erlauben.

WO 93/24086 beschreibt einen Tupfer, dessen saugfähiges Textilteil aus unterschiedlichen Lagen textilen Materials besteht und der einen Röntgenkontrastfaden aufweist. Die Mehrlagigkeit des Textilteils führt zu einem relativ starken Tupfer und macht ihn für neurochirurgische und mikrochirgische Operationen ungeeignet

Aufgabe der Erfindung ist die Schaffung eines medizinischen Hilfsmittels zur verbesserten Blutstillung bei Blutungen.

Erfindungsgemäß wird die Aufgabe durch die Verwendung von Tranexamsäure für die Präparation oder Herstellung medizinischer Tupfer, Kompressen oder Pflaster gelöst.

Die Erfindung umfasst auch medizinische Tupfer, Kompressen oder Pflaster, bei denen mindestens der zur Wundauflegung bestimmte Teil Tranexamsäure enthält und/oder freisetzt.

Bei Verwendung von Lösungen von Antifibrinolytika sind die getränkten medizinischen Tupfer oder Kompressen gegen Austrocknung geschützt verpackt.

Überraschend hat sich herausgestellt, dass ein mit dem Antifibrinolytikum Tranexamsäure getränkter Tupfer geeignet ist, bei chirurgischen Operationen eine Blutstillung herbeizuführen und damit zu wesentlich verbesserten Operationsbedingungen beizutragen.

Antifibrinolytika werden in bekannter Weise bei Hyperfibrinolyse, d.h. gesteigerter Fibrinolyse infolge vermehrter Bildung von Plasminogen als Injektionslösung oder Filmtabletten oral appliziert. Sie sollen einem Fibrinogenmangel infolge Thrombosebildung entgegenwirken. Eine topische Anwendung bei Wunden zur Wundstillung ist nicht bekannt.

Der maximale Effekt der Gabe von Antifibrinolytika, wie zum Beispiel bei Injektion von Tranexamsäure wird nach ca. 6 Stunden erreicht von Nebenwirkungen, wie Nausea, Vomitus oder Diarrhoe begleitet, wobei die Nebenwirkungen bei Absinken der Dosis wieder zurückgehen.

In der Erfindung wird als Antifibrinolytikum Tranexamsäure verwendet und die erfindungsgemäßen Tupfer, Kompressen oder Pflaster enthalten Tranexamsäure.

Zur Erfindung gehören auch medizinische Hilfsmittel zur Blutstillung, bestehend aus einem chirurgischem Tupfer oder Kompresse und einer Lösung von Tranexamsäure für die Tränkung des Tupfers oder der Kompresse vor Applikation.

Mit den mit Antifibrinolytika versehenen medizinische Tupfern wird nicht nur die Operationszeit verringert. Die schneller erreichbare Blutstillung führt zu einem verbessertem medizinischen Verlauf der Operation mit verringerten Komplikationen durch schwerer beinflußbare Blutkoagulation bei übermäßiger Blutung während der Operation. Bereits durch die geringere Operationszeit wird allein schon dadurch das Infektionsrisiko verringert. Verbessert wird auch die postoperative Hämostase.

Anhand nachfolgender Ausführungsbeispiele wird die Erfindung näher erläutert:

### Ausführungsbeispiel 1

Medizinische Tupfer, deren Textilteil mit Tranexamsäurelösung getränkt ist, sind in einer Folienpackung steril verschlossen und gegen Austrocknung geschützt. Die Folienpackung wird vor dem Einsatz an der Wunde geöffnet.

Der getränkte Tupfer wird mit seinem Textilteil auf zu stillende Bereiche der Operationsstelle aufgelegt. Überraschend wird mit den getränkten Tupfern eine Wundstillung innerhalb kurzer Zeit erreicht Waren mit herkömmlichen, mit physiologischer Kochsalzlösung getränkten Tupfern etwa 60 bis 80 Tupfer zur Blutstillung erforderlich, wurde eine Blutstillung mit dem mit Tranexamsäure getränkten Tupfer bereits mit 20 bis 40 Stück erreicht. Gleichzeitig wurde die Anwendung einer bipolaren Pinzette zur Verödung von Gefäßen von bisher 20 bis 40 zu verödendenden Gefäßen auf 2 bis 4 reduziert.

Mit den mit Tranexamsäurelösung getränkten Tupfern wird nicht nur die Operationszeit verringert. Die schneller erreichbare Blutstillung führt zu einem verbessertem medizinischen Verlauf der Operation mit verringerten Komplikationen durch schwerer beinflußbare Blutkoagulation bei übermäßiger Blutung während der Operation. Verbessert wird auch die postoperative Hämostase.

Überraschend hat sich gezeigt, dass der erfindungsgemäße Tupfer bei der Operation nicht nur gut handhabbar ist, sondern auch einen positven Einfluß auf die Wundheilung ausübt.

### Ausführungsbeispiel 2

Eingesetzt wird eine Packung aus chirurgischem Tupfer und Ampullen mit Tranexamsäurelösung (Konzentration: 100 mg Tranexamsäure auf 1 ml Wasser). Unmittelbar vor Einsatz an der Operationsstelle bei einer neurochirurgischen Operation wird der Textilteil des chirurgischen Tupfer in einer wässrigen Tranexamsäurelösung getränkt. Der mit Tranexamsäure getränkte Tupfer wird auf die Wundstelle aufgelegt. Innerhalb kurzer Zeit tritt Wundstillung ein.

Sowohl Tränkung als auch die Anwendung an der Operationsstelle sind unkomplizierte und schnell durchzuführende Handlungen. Gegenüber bisher bekannten Maßnahmen zur Blutstillung, die zum Teil einen Zeitraum von etwa 30 min erfordern, wird eine wesentlich schnellere lokale Blutstillung erreicht.

## Patentansprüche

1. Verwendung von Tranexamsäure als alleinigen aktiven Wirkstoff zur Präparation oder Herstellung medizinischer Tupfer, Kompressen oder Pflaster **zur schnellen lokalen Blutstillung.**

2. Vervendung nach Anspruch 1 **dadurch gekennzeichnet, dass** mindestens der zur Wundauflegung bestimmte Teil Tranexamsäure enthält und/oder freisetzt.

3. Verwendung nach Anspruch 2, **dadurch gekennzeichnet, dass** der mit Tranexamsäure getränkte Tupfer oder die getränkte Kompresse oder das getränkte Pflaster steril und gegen Austrocknung geschützt verpackt sind.

4. Verwendung von einem medizinischen Tupfer, einer Kompresse oder einem Pflaster und einer Tranexamsäurelösung für die Tränkung des Tupfer, der Kompresse oder des Pflasters vor Applikation zur Herstellung eines medizinischen Hilfsmittels zur Schnellen lokalen Blutstillung.

## Claims

1. Use of tranexamic acid as the sole active ingredient for preparing sponges, compresses or patches for fast local hemostasis.

2. Use according to claim 1, wherin at least the part applied to the wound contains and/or releases tranexamic acid.

3. Use according to claim 2, wherin the sponge impregnated with tranexamic acid or the compress impregnated with tranexamic acid or the patch impregnated with tranexamic acid are packaged sterilely and protected against dehydration.

4. Use of a medical sponge, a compress or a patch and a tranexamic acid solution for impregnating the sponge, compress oder patch before application for preparing a medical device for fast local hemostasis.

## Revendications

1. Utilisation de l'acide tranexamique comme seul agent actif pour la préparation de tampons, compresses ou pansements médicaux pour l'hémostase locale rapide.

2. Utilisation selon la revendication 1, **caractérisée en ce qu'**au moins la partie destinée à couvrir la plaie contient et/ou libère de l'acide tranexamique.

3. Utilisation selon la revendication 2, **caractérisée en ce que** le tampon imprégné d'acide tranexamique ou la compresse imprégnée ou le pansement imprégné sont emballés de manières stérile et protégés contre le dessèchement.

4. Utilisation d'un tampon, d'une compresse ou d'un pansement médical et d'une solution d'acide tranexamique pour l'imprégnation du tampon, de la compresse ou du pansement avant application, pour préparer un produit auxiliaire médical pour l'hémostase locale rapide.
